## Europäisches Patentamt

## European Patent Office

## Office européen des brevets

(19)

(11) Veröffentlichungsnummer: **0 018 496**
**B1**

(12) ## EUROPÄISCHE PATENTSCHRIFT

(45) Veröffentlichungstag der Patentschrift:
**06.11.85**

(51) Int. Cl.⁴: **A 61 F 2/00,** A 61 L 17/00,
A 61 B 17/56

(21) Anmeldenummer: **80101583.5**

(22) Anmeldetag: **26.03.80**

(54) Chirurgisches Netzwerk.

(30) Priorität: **28.04.79 DE 2917446**

(43) Veröffentlichungstag der Anmeldung:
**12.11.80 Patentblatt 80/23**

(45) Bekanntmachung des Hinweises auf die Patenterteilung:
**06.11.85 Patentblatt 85/45**

(84) Benannte Vertragsstaaten:
**AT BE CH DE FR GB IT NL SE**

(56) Entgegenhaltungen:
**CH - A - 495 755**
**DE - A - 2 827 289**
**FR - A - 2 088 548**
**FR - A - 2 278 348**
**FR - A - 2 361 092**
**FR - A - 2 394 624**
**FR - A - 2 409 278**
**US - A - 4 064 567**
**US - A - 4 141 087**

(73) Patentinhaber: **Merck Patent Gesellschaft mit beschränkter Haftung, Frankfurter Strasse 250, D-6100 Darmstadt (DE)**

(72) Erfinder: **Draenert, Klaus, Dr., Uhlandstrasse 16, D-8012 Ottobrunn (DE)**

(74) Vertreter: **Schüttler, Reinhard, Dr., Merck Patent GmbH Postfach 4119, D-6100 Darmstadt 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein chirurgisches Netzwerk zur Armierung von bei der Implantation von Endoprothesen verwendetem Knochenzement, wobei das Netzwerk eine lichte Maschenweite von etwa 1–10 mm aufweist, aus Fäden aus Polymeren mit einer Dicke von 50–300 µm und ggf. aus nichtresorbierbaren Fäden oder Drähten mit einer Dicke von 100-750 µm besteht und in Form eines einseitig geschlossenen Köchers ausgebildet ist.

Es sind eine Reihe von im Körper resorbierbaren oder nicht resorbierbaren Materialien bekannt, die in der Chirurgie Verwendung finden. Sie können z.B. in Form von Fäden oder Drähten als resorbierbares oder nicht resorbierbares Nahtmaterial oder in Form von Platten, Filmen, Stiften oder Schrauben zur Stützung oder Verbindung von Skeletteilen verwendet werden. Es ist z.B. in der FR–A–2088548 auch schon vorgeschlagen worden, resorbierbares Fadenmaterial in Form von gewebten, geflochtenen oder gewirkten textilen Strukturen zur Behebung von Brüchen, zur Stützung der verletzten Leber, Niere oder anderer Organe oder zur Ausbesserung von Arterien, Venen oder anderer Leitungen zu verwenden. Ferner ist vorgeschlagen worden, resorbierbare Fäden in Form von kleingeschnittenen Häckseln handelsüblichen Knochenzementen beizumischen.

Solche handelsüblichen Knochenzemente auf Acrylat- beziehungsweise Methacrylatbasis werden verwendet zur Auffüllung von Knochendefekten, bei sogenannten Verbundosteosynthesen oder bei der Implantation von Endoprothesen. Durch die Einarbeitung von resorbierbaren Fadenstücken bei der Implantation von Endoprothesen soll erreicht werden, dass nach deren Resorption in dem nicht resorbierbaren Knochenzement Porositäten zum Einwachsen von Körpergewebe geschaffen werden. Auf diese Weise soll eine verbessserte Langzeitstabilität der Prothese erreicht werden.

Ein Einwachsen von körpereigenem Gewebe ist jedoch hierbei nur dort möglich, wo zufällig ein solches Polymerfadenstück bis an die Oberfläche des Zementes reicht und von dort resorbiert werden kann. Die Fadenstücke, die im Inneren des Zementes liegen, können dagegen von Körpersäften nicht erreicht und resorbiert werden und tragen daher nicht zu einer Verwachsung der Prothese mit Körpergewebe bei. Es kann also erwartet werden, dass die Langzeitstabilität einer Prothese, die mit Hilfe eines mit Häckseln von resorbierbaren Polymerfäden versetzten Knochenzementes implantiert ist, bestenfalls geringfügig verbessert ist. Da überdies die eingearbeiteten Fadenstücke relativ kurz sein müssen, um überhaupt mit dem Zement gemischt werden zu können, ist davon auch keine verbesserte Elastizität und Stabilität des um die implantierte Prothese gebildeten Zementköchers zu erwarten. Der Zementköcher ist nämlich in über 60% der Lockerungen von Femurschaftprothesen und Pfannenimplantaten den mechanischen Beanspruchungen nicht gewachsen. In den meisten Fällen wird der Zementköcher durch den Metallschaft bzw. durch die Kunststoffpfanne gesprengt oder frakturiert.

In der US–A 4 064 567 wird ein chirurgisches Material der eingangs genannten Art beschrieben, das aus nichtresorbierbaren Fäden oder Drähten besteht, die ein Netzwerk bilden. Das Material dient zur Stabilisierung von bei der Implantation von Prothesen verwendetem Knochenzement. Das Netzwerk kann in der Form eines einseitig geschlossenen Köchers vorliegen, in den z.B. eine Femurschaftprothese eingeschoben werden kann. Durch dieses Material kann der um die Prothese gebildete Zementköcher zwar zunächst gut stabilisiert werden, es erfolgt jedoch keine Langzeitstabilisierung durch ein Verwachsen des Implantats mit dem umgebenden Knochen.

Es bestand deshalb die Aufgabe, ein chirurgisches Material zu schaffen, das bei der Implantation von Endoprothesen eingesetzt werden kann und das sowohl unmittelbar zur Verbesserung der Elastizität und Stabilität beiträgt als auch eine hervorragende Langzeitstabilität garantiert.

Diese Aufgabe wurde durch die vorliegende Erfindung gelöst.

Es wurde nämlich gefunden, dass die Einarbeitung des im Anspruch 1 genannten Netzwerks in den Knochenzement zu einer wesentlich verbesserten Kurz- und Langzeitstabilität führt, wenn das Netzwerk zumindest teilweise aus im Körper resorbierbaren organischen Polymeren besteht.

Gegenstand der Erfindung ist demgemäss ein chirurgisches Netzwerk zur Armierung von bei der Implantation von Endoprothesen verwendetem Knochenzement, wobei das Netzwerk eine lichte Maschenweite von etwa 1 bis 10 mm aufweist, aus Fäden aus Polymeren mit einer Dicke von 50–300 µm und ggf. aus nichtresorbierenden Fäden oder Drähten mit einer Dicke von 100–750 µm besteht und in Form eines einseitig geschlossenen Köchers ausgebildet ist, und wobei das Netzwerk dadurch gekennzeichnet ist, dass zumindest ein Teil der Fäden des Netzwerks aus resorbierbaren organischen Polymeren, einschliesslich natürlicher Polymere wie Catgut oder Kollagen, besteht.

Die erfindungsgemässen Materialien haben den Vorteil, dass durch das Netzwerk sofort eine sehr starke Stabilisierung des Sitzes der implantierten Endoprothese wird. Dazu genügt es an sich schon, wenn nur eine Lage des erfindungsgemässen Netzwerkes verwendet wird. In der Regel wird man jedoch das erfindungsgemässe Material in Form von mehreren, vorzugsweise etwa 2-3, aufeinanderfolgenden Lagen verwenden.

Während die Stabilisierung des Sitzes der implantierten Endoprothesen unmittelbar nach Einbringen des erfindungsgemässen Materials in den Körper durch den Verbund des noch intakten Netzwerks gewährleistet ist, wird diese Stabilisierung im Falle eines Netzwerkes aus resorbierbaren organischen Polymeren mit fortschreitender Resorption der organischen Polymere durch das gleichzeitig in die entstehenden Hohlräume einwachsende körpereigene Gewebe erreicht. Durch die gewählten Dimensionen des erfindungsgemässen resorbierbaren Materials mit einer Fadendicke von etwa 50 – etwa 300 μm, vorzugsweise etwa 80 – etwa 200 μm, und der Maschenweite etwa 1 bis etwa 10 mm, vorzugsweise etwa 2 bis etwa 5 mm, ist gewährleistet, dass in den nicht resorbierbaren Knochenzement ein Hohlraumsystem in für das Knochenwachstum sehr vorteilhaften Dimensionen entsteht, das in dem Masse, in dem das Netzwerk resorbiert wird, durch tragfähiges Knochengewebe gefüllt wird.

Die Stabilisierung des Sitzes der implantierten Endoprothesen kann zusätzlich noch dadurch beeinflusst werden, dass bei einem mehrlagigen Material eine Lage aus einem nicht resorbierbaren Material besteht, das also seinen Verbund unabhängig von der Resorption der übrigen Lagen beibehält und so zusätzlich zur Dauerstabilität beiträgt. Dazu können z.B. nicht resorbierbare organische Polymerfäden wie z.B. Nylonfäden verwendet werden. Als besonders vorteilhaft hat es sich jedoch erwiesen, wenn eine Lage aus einem körperverträglichen und im Körper nicht angegriffenen Netzwerk aus Metallfäden besteht. Als Material für die Metallfäden ist z.B. Titan oder eine Legierung aus Cobalt, Chrom und Molybdän geeignet.

Die Verwendung zumindest einer Lage eines Netzwerkes aus Metallfäden bringt neben einer sehr guten Stabilisierung auch den Vorteil, dass jederzeit eine sehr leichte röntgenographische Kontrolle des behandelten Knochendefektes möglich ist.

Als resorbierbare organische Polymere können an sich alle bisher schon als resorbierbare chirurgische Materialien bekannten Stoffe verwendet werden. Neben der Resorbierbarkeit ist lediglich Voraussetzung, dass sie zu dem erfindungsgemässen Netzwerk gestaltet werden können. Es kommen dabei sowohl natürliche Materialien wie z.B. Catgut oder Kollagen in Frage, als auch synthetische Polymeren, wie z.B. Polyglycolide, Polylactide oder Mischpolymerisate aus Lactid und Glycolid. Verwendet werden können auch Polyaminosäuren. Diese Materialien sind bekannt oder können nach bekannten Methoden hergestellt werden. Dabei können auch die Fäden in beliebiger Dicke hergestellt werden. Auch das Verbinden der Fäden zu dem erfindungsgemässen Netzwerk geschieht in bekannter Weise durch Weben, Stricken, Flechten, Wirken, Kleben oder Schweissen. Bei der Implantation von Endoprothesen besitzt die einseitig geschlossene Röhrenoder Köcherform sehr grosse Vorteile. Bei der Implantation einer Hüftgelenkprothese traten bisher nämlich eine Reihe von Schwierigkeiten auf, die mit Hilfe des erfindungsgemässen Materials überwunden werden können. So musste bisher sehr grosse Sorgfalt verwendet werden, z.B. durch Anwendung spezieller Zielgeräte, um den Prothesenschaft genau zentriert in den Oberschenkelknochen einzuführen und so einen direkten Kontakt der Prothese zum Knochen zu vermeiden. Auch kam es häufig vor, dass beim Einführen der Prothese Knochenzement in den Markraum eingepresst wurde.

Bei der Verwendung eines Köchers aus dem erfindungsgemässen Material, der im einfachsten Fall durch einseitiges Zubinden einer Röhre gewonnen werden kann, wird zunächst dieser Köcher z.B. mit Hilfe des Prothesenschafts in den vorbereiteten Oberschenkelknochen eingeführt und nach Entfernen der Prothese mit dem noch plastisch verformbaren Knochenzement gefüllt. Wenn nun die Prothese eingeführt wird, wobei kein Zielgerät erforderlich ist, da der erfindungsgemässe Köcher den Prothesenschaft zentriert, wird durch den dabei auftretenden Druck das erfindungsgemässe Netzwerk bis an das noch intakte Knochengewebe gepresst und gleichzeitig werden die zwischen den Fäden verbleibenden Hohlräume mit Knochenzement gefüllt, der auf diese Weise ebenfalls an das lebende Gewebe angrenzt. Das Einpressen von Knochenzement in den unterhalb der Prothese angrenzenden Markraum wird dabei durch den unten geschlossenen Köcher weitestgehend verhindert. Durch die Verwendung eines resorbierbaren Fadenmaterials wird dann nach der Implantation das resorbierbare Material vom Rand her resorbiert und die entstehenden Hohlräume werden gleichzeitig mit einem Knochengerüst gefüllt. Durch die vorzugsweise mehrlagige Ausbildung des erfindungsgemässen Materials entsteht so ein dreidimensionales Kanalsystem in dem nicht resorbierbaren Knochenzement, das mit einem tragfähigen Knochengerüst gefüllt ist.

Durch den Einbau des erfindungsgemässen Netzwerkes aus resorbierbarem Material in Verbindung mit einer oder mehreren Lagen eines Metallnetzes wird die Stabilität des um die Prothese gebildeten Köchers so stark erhöht, dass eine Sprengung des Zementköchers durch den Prothesenschaft bzw. durch die Pfannenprothese kaum noch zu befürchten ist.

Bisweilen kommt es bei solchen Implantationen vor, dass wegen postoperativer Störungen die Prothese wieder entfernt werden muss. Dazu musste bisher der Zementköcher Stück für Stück mühsam herausgeschlagen werden, wobei es unvermeidbar war, dass auch beträchtliche Teile des angrenzenden Knochengewebes in Mitleidenschaft gezogen wurden. Durch die Einarbeitung des erfindungsgemässen Netzwerkes, insbesondere eines, das zumindest eine Lage eines nichtresorbierbaren, insbesondere metallischen Netzwerkes enthält, wird der Zementköcher jedoch so stabilisiert, dass er, falls notwendig, als Ganzes entfernt werden kann. Hierfür können zusätzlich am offenen Ende des Köchers noch metallene Laschen zur Extraktion angebracht werden.

Die erfindungsgemässen Materialien werden zusammen mit einem der üblichen Knochenzemente verarbeitet. Dies sind autopolymerisierende Stoffe, die unmittelbar vor der Operation angerührt werden und in plastischer Form in den Körper eingebracht werden und dort innerhalb weniger Minuten aushärten. Solche Knochenzemente auf Acrylat- bzw. Methacrylatbasis, sind z.B. unter dem Warenzeichen Palacos® im Handel. Sie werden so zubereitet, dass etwa 2 Teile einer feinteiligen, einen Polymerisationskatalysator (z.B. Dibenzoylperoxid) enthaltenden Präpolymerisats mit einem Teil eines flüssigen Monomeren, das einen Beschleuniger (z.B. Dimethylp-toluidin) enthält, zu einer formbaren Masse gemischt werden, die in den Körper implantiert wird und dort aushärtet. Als Präpolymerisat dient insbesondere Polymethylmethacrylat oder ein Mischpolymerisat aus Methylacrylat und Methylmethacrylat. Als Monomeres wird z.B. Acrylsäure- oder Methacrylsäuremethylester oder deren Gemische verwendet. Besonders bevorzugt werden die erfindungsgemässen Materialien mit den in der EP-A 0 016 906 beschriebenen Implantationsmaterialien auf Basis von Polyacrylaten verarbeitet, die zusätzlich etwa 5 bis etwa 35 Gew.% resorbierbares Tricalciumphosphat mit einer Teilchengrösse zwischen 50 und 300 µm enthalten. (Die EP-A 0 016 906 ist Stand der Technik entsprechend Artikel 54(3)EPU.)

Das in diesen Implantationsmaterialien enthaltene Tricalciumphosphat dient einmal zur Stimulierung des Knochenwachstums, da Calciumphosphat ein wesentlicher Bestandteil des natürlichen Knochens ist, zum anderen werden bei der Resorption des Tricalciumphosphats weitere Hohlräume geschaffen, in die Knochengewebe einwachsen kann und die auf diese Weise für eine noch bessere Verzahnung des Implantats mit der Umgebung sorgen.

Die wesentlichen Vorzüge des erfindungsgemässen Netzwerks werden jedoch auch bei einer Verarbeitung mit anderen Knochenzementen deutlich, so dass die erfindungsgemässen Materialien mit allen üblichen Knochenzementen verarbeitet werden können.

Das erfindungsgemässe Netzwerk kann aus Polymerfäden hergestellt werden, die mit feinteiligem Tricalciumphosphat bestückt sind. Diese Bestückung mit Tricalciumphosphat kann z.B. so erfolgen, dass das Flechten der zunächst sehr feinen Polymerfäden zu einem Faden der erfindungsgemässen Dicke in Gegenwart von Tricalciumphosphat-Pulver durchgeführt wird, wobei Tricalciumphosphat Partikel mit in den Faden verflochten werden. Die Bestückung kann jedoch auch so erfolgen, dass die Polymerfäden oberflächlich angelöst oder geschmolzen werden und das Tricalciumphosphat so mit der Oberfläche verklebt wird. Die so mit Tricalciumphosphat bestückten resorbierbaren Polymerfäden können dann auf übliche Weise zu dem erfindungsgemässen Netzwerk verbunden werden. Ein derartig mit Tricalciumphosphat bestücktes Netzwerk ist damit auch in der Lage, das Knochenwachstum in der Umgebung zu stimulieren.

Durch die Erfindung steht den Chirurgen ein Material zur Verfügung, das bei der Implantation von Endoprothesen sehr vorteilhaft eingesetzt werden kann.

Beispiel 1

100 Meter eines chirurgischen Nahtmaterials, das unter dem Namen Catgut im Handel ist (Fadendicke ca. 120 µm), wird auf einer Rundstrickmaschine zu einem endlosen Strumpf mit einer Maschenweite von etwa 2 auf 4 mm und einer Gesamtweite von 14 Maschen verstrickt. Durch Aufziehen des Strumpfes auf einen Zylinder und Umstülpen wird ein zweilagiger Strumpf erhalten, durch zweimaliges Umstülpen ein vierlagiger Strumpf.

Beispiel 2

Ein resorbierbares chirurgisches Nahtmaterial auf Basis von Polylactiden (Fadendicke ca. 100 µm), das bei einer Temperatur oberhalb 200°C zu schmelzen beginnt, wird bei dieser Temperatur mit Tricalciumphosphatpartikeln einer Korngrösse von 100–200 µm bestückt. Ein solcher Faden kann analog Beispiel 1 zu einem zylinderförmigen Strumpf mit einer lichten Maschenweite von 2–4 mm verstrickt werden, aus dem durch mehrmaliges Ineinanderstülpen ein mehrlagiger zylinderförmiger Strumpf erhalten wird.

Beispiel 3

Ein chirurgisches Drahtmaterial mit einer Dicke von 150 µm wird auf einer Rundstrickmaschine zu einem zylindrischen Strumpf mit einer Maschenweite von 2 bis 4 mm und einer Gesamtweite von 14 Maschen verstrickt. Dieser Strumpf wird auf einem Hohlzylinder aufgezogen und in einen Hohlzylinder entsprechend grösseren Umfangs eingeschoben, auf den ein Strumpf aus resorbierbarem Material nach Beispiel 1 aufgezogen ist. Nach Entfernen der Hohlzylinder erhält man einen zylindrischen, zweilagigen Strumpf, bei dem eine Lage eines nicht resorbierbaren metallischen Netzwerks von einer Lage eines resorbierbaren Netzwerks bedeckt ist. Durch Ineinanderstülpen kann z.B. ein vierlagiger Strumpf erhalten werden, wobei auf eine äussere, resorbierbare Lage zwei nicht resorbierbare und noch eine resorbierbare Lage folgen.

Beispiel 4

Eine Femurschaftprothese wird mit einem nach Beispiel 1 hergestellten mehrlagigen Strumpf überzogen und in eine dafür vorbereitete Markhöhle am proximalen Femur eingeführt. Nach Herausziehen der Prothese, wobei der Strumpfköcher in der Markhöhle verbleibt, wird dieser mit einem üblichen Knochenzement gefüllt und anschliessend wird die Prothese in korrektem Winkel vorsichtig in den Strumpfköcher eingeführt. Dabei wird der Zement nach allen Seiten durch die Maschen des Strumpfes hindurch gepresst. Die Prothese wird bis zum Aushärten des Zementes unter Druck implantiert gehalten, so dass ein Herausdrücken des Schaftes auf Grund der Polymerisationsschrumpfung des Kunststoffes verhindert wird. Nach Aushärten

des Zementes ist die Prothese stabil implantiert und kann nicht mehr herausgeschlagen werden. Bei dem Versuch, eine solche Prothese mit Gewalt zu entfernen, kommt es entweder zur Frakturierung des knöchernen Köchers oder zum Herausreissen der Prothese mitsamt dem Zementköcher, wobei die Knocheninnenfläche entsprechend verletzt wird.

In gleicher Weise kann ein nach Beispiel 2 oder Beispiel 3 hergestelltes mehrlagiges Material verwendet werden.

Beispiel 5

Ein arthrotisch verändertes Hüftgelenk wird eröffnet, der Schenkelhals freipräpariert, die Kapsel entfernt und der Hüftkopf mit dem Schenkelhals 1 cm oberhalb des Trochanter minors mit einem Winkel zur Horizontalebene von 40 Grad reseziert. Die Pfannenhöhle wird von Knorpel befreit, das Labrum acetabulare entfernt und die Fossa acetabuli ausgeräumt. Nach sorgfältiger Blutstillung werden im Pfannendach vier 10 mm – 16mm im Durchmesser messende zylindrische Verankerungslöcher angebracht. Ein nach Beispiel 1 angefertigter Strumpf wird in mehreren Schichten übereinander gestülpt und in das getrocknete Pfannenbett gelegt. Sodann wird mit dem Präparationstupfer das Maschenwerk in die Verankerungslöcher im Pfannendach hineingedrückt. Nach sorgfältiger Austrocknung des gesamten Bettes und Anbringen eines Entlüftungsloches zum lateralen Pfanneneck, das die Tiefe der Verankerungslöcher drainiert, wird der Knochenzement eingebracht und mit dem Pfannenimplantat fest in die vorbereitete Pfannenhöhle in korrekter Position imprimiert. Unter voller Ausnützung der druckaufnehmenden Fläche im Pfannendach wird die Prothese unverrückbar bis zur Aushärtung des Zementes eingepresst gehalten. Nach dieser Zeit ist die Pfannenprothese stabil implantiert und kann nicht mehr herausgeschlagen werden.

**Patentansprüche**

1. Chirurgisches Netzwerk zur Armierung von bei der Implantation von Endoprothesen verwendetem Knochenzement, wobei das Netzwerk eine lichte Maschenweite von etwa 1 – 10 mm aufweist, aus Fäden aus Polymeren mit einer Dicke von 50–300 µm und ggf. aus nicht-resorbierbaren Fäden oder Drähten mit einer Dicke von 100–750 µm besteht und in Form eines einseitig geschlossenen Köchers ausgebildet ist, dadurch gekennzeichnet, dass zumindest ein Teil der Fäden des Netzwerks aus resorbierbaren organischen Polymeren, einschliesslich natürlicher Polymere wie Catgut oder Kollagen, besteht.

2. Netzwerk nach Anspruch 1, dadurch gekennzeichnet, dass es mehrlagig ausgeführt ist.

3. Netzwerk nach den Ansprüchen 1 oder 2, dadurch gekennzeichnet, dass die resorbierbaren Polymerfäden mit Tricalciumphosphat bestückt sind.

**Claims**

1. Surgical network material for the reinforcement of bone cement used in the implantation of endoprostheses, whereby the network material has an internal mesh width of abouth 1 – 10 mm, consists of filaments of polymers with a thickness of 50 – 300 µm, and, if the occasion arises, of non-resorbable filaments or wires with a thickness of 100 – 750 µm, and is formed in the shape of a one-sided closed socket, characterised in that at least a part of the filaments of the network material consists of a resorbable organic polymer, including natural polymers like catgut or collagen.

2. Network material according to claim 1, characterised in that it is in the form of a multi-layer material.

3. Network material according to claims 1 or 2, characterised in that the resorbable polymer filaments are provided with tricalcium phosphate.

**Revendications**

1. Treillis chirurgical pour armer le ciment d'os utilisé lors de l'implantation d'endoprothèses, dans lequel le treillis présente une maille libre d'environ 1 à 10 mm, en fils de polymères d'une épaisseur de 50-300 µm et le cas échéant en fils ou filaments non-résorbables d'une épaisseur de 100-750 µm et qui est constitué d'un carquois fermé à une extrémité et dans lequel le treillis est caractérisé en ce que au moins une partie des fils du treillis est constituée de polymères organiques résorbables, y compris des polymères naturels comme du catgut ou du collagène.

2. Treillis selon la revendication 1, caractérisé en ce qu'il est réalisé en structure multicouche.

3. Treillis selon les revendications 1 ou 2 caractérisé en ce que les fils polymères résorbables sont garnis de phosphate tricalcique.